# EUROPÄISCHE PATENTSCHRIFT

(11) **EP 3 164 164 B1**
(45) Veröffentlichungstag und Bekanntmachung des Hinweises auf die Patenterteilung: **11.03.2026**
(21) Anmeldenummer: 15739497.4
(22) Anmeldetag: 03.07.2015
(51) Int. Cl.: A61L 9/12

(54) **VORRICHTUNG ZUR ERZEUGUNG EINES DUFTSTOFFBELADENEN FLUIDSTROMES**
APPARATUS FOR GENERATING A FRAGRANCE-LADEN FLUID STREAM
DISPOSITIF POUR GÉNÉRER UN FLUX DE FLUIDE CHARGÉ DE PARFUM

(30) Priorität: 04.07.2014 DE 202014005446 U
(43) Veröffentlichungstag der Anmeldung: 10.05.2017
(73) Patentinhaber: CWS-boco International GmbH, 47119 Duisburg (DE)
(72) Erfinder: KÄPPELI, Roland, 5634 Merenschwand (CH); SCHEIWILLER, Felix, 9444 Diepoldsau (CH)
(74) Vertreter: Lusuardi, Werther
(86) Internationale Anmeldenummer: PCT/CH2015/000096
(87) Internationale Veröffentlichungsnummer: WO 2016/000086

(56) Entgegenhaltungen:
- EP-A1- 0 890 365
- WO-A1-2006/061803
- FR-A1- 2 100 527
- US-A1- 2003 150 874
- US-A1- 2007 181 706
- US-A1- 2007 210 101
- US-A1- 2011 072 711

## Beschreibung

Die Erfindung bezieht sich auf einen Vorrichtung zur Erzeugung eines duftstoffbeladenen Fluidstromes gemäss dem Oberbegriff des Anspruchs 1.

Eine Vorrichtung zur Erzeugung eines duftstoffbeladenen Fluidstromes ist aus dem Dokument EP 0 890 365 B1 bekannt. Diese bekannte Vorrichtung weist jedoch die Nachteile auf, dass der Verbrauch von Geruchsstoff nicht optimiert ist und zudem die Wartung, die Reinigung und das Nachfüllen der Duftstoffe ebenfalls nicht optimiert sind.

Aus der US-A 2007/181706 Yeh et al. ist eine Duftstoff spendende Uhr bekannt, mit einem Hohlraum, in welchem der Duftstoffbehälter durch erste und zweite Perforationsmittel perforierbar ist. Die Perforationsmittel sind nicht relativ zum Hohlraum bewegbar und nehmen nur eine einzige Position ein.

Aus der FR 2 100 527 Malczewski ist eine Desinfektionsvorrichtung bekannt, welche aber keinen Hohlraum zur Aufnahme eines Duftstoffbehälters aufweist.

Aus der US-A 2011/072711 Black et al. ist eine Ampulle zur Aufbewahrung und Dispersion einer flüchtigen Flüssigkeit bekannt.

Aus der US-A 2014/0183273 Hafer et al. ist eine Vorrichtung zur Aktivierung eines eine flüchtige Substanz enthaltenden Reservoirs bekannt.

Aus der US 2003/0150874 Meire ist schliesslich eine Düse bekannt, zur Versprühung einer aktiven Substanz mit einem Düsenkopf und einem den Düsenkopf aufnehmenden Düsengehäuse.

Her will die Erfindung Abhilfe schaffen. Der Erfindung liegt die Aufgabe zugrunde, eine Vorrichtung zur Erzeugung eines duftstoffbeladenen Fluidstromes bereitzustellen, die den Duftstoff verbrauchsoptimiert einsetzt und in vereinfachter Weise wartbar, reinigbar und auffüllbar ist.

Die Erfindung löst die gestellte Aufgabe mit der Vorrichtung zur Erzeugung eines duftstoffbeladenen Fluidstromes, welcher die Merkmale des Anspruchs 1 aufweist.

Die durch die Erfindung erreichten Vorteile sind im Wesentlichen darin zu sehen, dass dank des erfindungsgemässen Gegenstandes des Anspruchs 1 der Verbrauch an Geruchsstoff minimiert ist und die Wartung, die Reinigung sowie die Nachfüllung von Wirkstoff und Verdunstungselementen vereinfacht ist. Gemäss dem Gegenstand des Anspruchs 1 sind insbesondere nachfolgende Punkte vereinfacht bzw. verbessert:

### Verbesserte Innenhygiene

Da die Rückstande der Wirkstoffe in den Behältnissen (Kartuschen) bleiben und nicht teilweise an den Wänden eines nicht auswechselbaren Verdunstungsraumes verbleiben, werden bei der Wartung der Vorrichtung gemäss Anspruch 1 Reinigungsschritte eingespart. Nicht genutzter, an den Wänden des Verdunstungsraumes haftender Wirkstoff wird mit dem Behältnis ausgewechselt, so dass die Hygiene optimiert ist und der Wartungsaufwand verringert wurde. Nach dem Stand der Technik bekannte Vorrichtungen erfordern eine gesonderte Reinigung des Verdunstungsraumes.

### Vermeidung des gesonderten Austausches von Verdunstungselementen

Die nach dem Stand der Technik bekannten Vorrichtungen erfordern das regelmässige Auswechseln von Verdunstungselementen, um die Funktionsfähigkeit des Geräts zu gewährleisten und um einen mikrobiellen Befall zu vermeiden. Sowohl geschultes als auch ungeschultes Personal wird bei diesem Schritt Schwierigkeiten und viel Zeit aufwenden müssen. Bei der Vorrichtung gemäss Anspruch 1 wird dieser Wartungsschritt durch die Verwendung von schnell austauschbaren Behältnissen vermieden, weil das Verdunstungselement im Behältnis angeordnet ist.

### Erleichterter, schnellerer Wechsel der Behältnisse mit Wirkstoffen/Duftträgern durch Vermeidung des Nachfüllens von Wirkstoff/Duftträgern

Darüber hinaus entfällt das Nachfüllen des Wirkstoffs. Nach dem Stand der Technik bekannte Vorrichtungen erfordern ein zeitaufwendiges Nachfüllen, was bei der Vorrichtung gemäss Anspruch 1 ebenfalls durch die Verwendung von Behältnissen vermieden werden kann.

### Vermeidung von Konservierungsmitteln

Zudem sind nach dem Stand der Technik Nachfüllflaschen erforderlich, die nach Öffnung mikrobiell angreifbar sind. Nach der Vorrichtung gemäss Anspruch 1 wird diese Problematik umgangen, da die Behältnisse für den Einmalgebrauch vorgesehen sind. Die Verwendung von Konservierungsmitteln kann dadurch vermieden werden. Zudem ist bei der Lagerung ein Verlustanteil der flüchtigen Wirkstoffe durch Verdampfung vermeidbar, weil die Wirkstoffe bei Lagerung in keiner Weise durch wiederholtes Öffnen bei der Lagerung von Nachfüllflaschen verloren gehen.

### Verkleinerung der Vorrichtungen

Ein weiterer Vorteil der erfindungsgemässen Vorrichtung ist darin zu sehen, dass die Vorrichtungen kleiner gestaltet werden können. Während wie oben erwähnt bei den nach dem Stand der Technik bekannten Vorrichtungen verschiedene Wartungsschritte wie das Nachfüllen der Wirkstoffe und das Austauschen der Verdunstungselemente eine gewisse Mindestgrösse des Gerätes erforderlich machen, kann die Vorrichtung nach dem Gegenstand des Anspruchs 1 aufgrund der leichten Handhabbarkeit wesentlich verkleinert werden bzw. miniaturisiert werden kann. Die erfindungsgemässen Vorrichtungen können aufgrund ihrer geringen Grösse versteckt im Raum angebracht werden und sind somit vor Vandalismus geschützt.

### Sofortiger Einsatz nach Wartung

Da in den Behältnissen permanent schon während der Lagerung eine Sättigungskonzentration vorliegt, ist die Vorrichtung zudem unmittelbar nach Perforierung der Behältnisse einsatzbereit, während bei den sich nach dem Stand der Technik bekannten Vorrichtung erst nach der Wartung eine Sättigungskonzentration einstellen muss, was über eine Stunde dauern kann.

Die im Anspruch 1 charakterisierte Vorrichtung basiert auf der natürlichen temperaturabhängigen Verdunstung des flüchtigen Wirkstoffs. Die Vorrichtung (12) hat vorzugsweise eine maximale Grundfläche von 1,5m x 1m und eine Höhe von maximal 1m. Weitere bevorzugte Ausführungsformen haben eine maximale Grundfläche von 0,9m x 0,5m und eine maximale Höhe von 50 cm. Besonders bevorzugte Ausführungsformen habe eine Grundfläche von 30 cm x 80 cm und eine Höhe von 40 cm. Stark verkleinerte Vorrichtungen gemäss Anspruch 1 haben vorzugsweise nur eine Grundfläche von maximal 6 cm x 5 cm und eine maximale Höhe von 5 cm. Diese verkleinerten Geräte können versteckt im Raum angebracht werden.

Der Hohlraum (4) ist vorzugsweise durch eine obere und untere Begrenzungsfläche umgrenzt (5; 6). Diese Begrenzungsflächen erleichtern die Fixierung der Behältnisse in dem Hohlraum (4). Vorzugsweise bietet der Hohlraum einem Behältnis Platz. In besonderen Ausführungsformen bietet der Hohlraum mehreren Behältnissen Platz. Der Hohlraum (4) hat vorzugsweise maximal eine Grundfläche von 50 cm x 50 cm und eine maximale Höhe von 60 cm. Der Hohlraum ist über eine Verbindung (22), die nicht die Austrittsöffnung (9) des Fluidkanals (2) ist, mit der Umgebungsluft verbunden.

Die Verbindung (22) zu der Umgebungsluft umfasst einen kann Luftkanals (3), der eine direkte Verbindung zwischen Umgebungsluft und Hohlraum darstellt. Neben dem Luftkanal (3) und dem Fluidkanal kann auch eine weitere Öffnung am Gehäuse vorliegen. Die Verbindung zwischen der Umgebungsluft (22) und dem Hohlraum kann auch über ein luftundichtes Gehäuse ausgestaltet sein (1).

In bevorzugten Ausführungsformen sind zwei, drei, vier oder mehr Hohlräume (4) mit entsprechenden Perforationsmitteln (18a, 18b) in der Vorrichtung vorhanden, wobei in besonderen Ausführungsformen die Anzahl der in der Vorrichtung platzierten Behältnisse (18) der Zahl der Hohlräume (4) entspricht. Bei Verwendung von zwei oder mehr Behältnissen (16) ist ein unterbrechungsfreier Betrieb möglich, da die Behältnisse in der Vorrichtung nacheinander (konsekutiv) geleert werden. Auf diese Weise lassen sich die Serviceintervalle verlängern.

Bei weiteren Ausführungsformen lassen sich durch parallele Nutzung von zwei oder mehr in der Vorrichtung platzierten Behältnissen verschiedene Wirkstoffe wie verschiedene Geruchsnoten oder Desodorantien miteinander kombinieren

Die ersten und zweiten Perforationsmittel (18a, 18b) können gemäss Anspruch 1 zwei Positionen (F.1) und F.2)) einnehmen. Bei Einnahme der ersten Position F.1) gemäss Anspruch 1 befinden sich die ersten und zweiten Perforationsmittel (18a, 18b) ausserhalb des Hohlraumes (4). In der zweiten Position F.2) befinden sich die ersten Perforationsmittel (18a), die Enden des Fluidkanals (8) und zweiten Perforationsmittel (18b) innerhalb des Hohlraumes (4) und sind in der Lage, ein im Hohlraum (4) befindliches Behältnis (16) zu perforieren.

Bei bevorzugten Ausführungsformen weist die Vorrichtung (12) ein Gehäuse (1) auf, wobei das Gehäuse mit den Perforationsmittel (18a, 18b) mechanisch gekoppelt ist. Bei Position F.1) der Vorrichtung gemäss Anspruch 17 befinden sich die ersten und zweiten Perforationsmittel (18a, 18b) nicht im Hohlraum. Bei geschlossenem Gehäuse ragen die Perforationsmittel (18a, 18b) in den Hohlraum hinein - dies entspricht dem Zustand F.2) des Gegenstandes des Anspruchs 1.

Die ersten und zweiten Perforationsmittel (18a, 18b) üben bei geschlossenem Gehäuse einen mechanischen Druck auf die Behältnisse aus, so dass eine Perforation der Behältnisse erfolgt.

Die Perforationsmittel (18a, 18b) sind vorzugsweise scharfkantig und bei bevorzugten Ausführungsformen als Schneidvorrichtungen ausgestaltet. Sie bestehen aus Metall, Kunststoff, Glas oder Material vergleichbarer Härte oder Kombinationen der genannten Materialien.

Die Perforationsmittel (18a, 18b) ragen nach erfolgter Perforation in das Behältnis (16) hinein und ermöglichen den Austritt der gesättigten Atmosphäre q* aus dem Behältnis (16) in den Fluidkanal (2). Die Perforationsmittel beschädigen hierbei nicht weitere im Behältnis angebrachte Trennwände (24), die das Behältnis in Verdunstungsraum (23) und Flüssigkeitskammer (25) kompartimentieren.

Bei bevorzugten Ausführungsformen befinden sich die ersten Perforationsmittel (18a) derart nah an den hohlraumseitigen Enden des Fluidkanals, dass bei der Perforierung des Behältnisses der überwiegende Teil der ausströmenden gesättigten Atmosphäre A*, vorzugsweise die gesamte Menge der ausströmenden gesättigten Atmosphäre A*, in den Fluidkanal eintritt. Bei weiteren bevorzugten Ausführungsformen sind die ersten Perforationsmittel (18a) identisch mit den hohlraumseitigen Enden (8) des Fluidkanals (2).

Die Perforation der ersten und zweiten Perforationsmittel (18a, 18b) kann im Ruhezustand zu einem moderaten Austritt des Wirkstoffs führen, so dass eine Grundbeduftung des Raumes erfolgt.

Bei bevorzugten Ausführungsformen sind die ersten Perforationsmittel (18a) an den hohlraumseitigen Enden des Fluidkanals (8) angebracht oder entsprechen diesen. Bei bevorzugten Ausführungsformen sind die zweiten Perforationsmittel (18b) an den Enden des Luftkanals (15) angebracht oder entsprechen diesen.

Die zweiten Perforationsmittel (18b) ragen ebenfalls bei geschlossenem Gehäuse (1) in den Hohlraum hinein. Wenn ein Behältnis im Hohlraum (4) platziert wird und das Gehäuse geschlossen wird, perforieren die zweiten Perforationsmittel (18b) an der vorgesehenen Stelle das Behältnis (16). Diese Perforation ergänzt die Perforation, die durch die ersten Perforationsmittel (18a) erzeugt wird. Zum Beispiel erfolgt durch die Perforation der zweiten Perforationsmittel (18b) bei Inbetriebnahme der Vorrichtung ein Druckausgleich im Behältnis. Die gesättigten Atmosphäre A* tritt im Ruhezustand oder bei Inbetriebnahme des Geräts kaum oder überhaupt nicht an der zweiten Perforationsstelle aus.

Die in die Hohlräume (4) einzusetzenden Behältnisse sind an mindestens einer Seite durch die Perforationsmittel (18a, 18b) perforierbar. Bei bevorzugten Ausführungsformen ist dies die Oberseite des Behältnisses (16). Die Stellen der Behältnisse, die von den Perforationsmitteln perforiert werden, sind vorzugsweise in Relation zu den übrigen Bereichen des Behältnisses dünner ausgestaltet. In besonders bevorzugten Ausführungsformen bestehen die Perforationsstellen der Behältnisse aus einer Folie, die aus einem Metall oder einem Kunststoff besteht. Es sind die Behältnisse verschliessende Folien (17). Perforierte Folien können nach Gebrauch wieder durch neue ersetzt werde, sodass die Behältnisse (16) optional wiederverwendbar sind. Vorzugsweise sind die Behältnisse (16) leicht handhabbare Kartuschen. Die Behältnisse sind in bevorzugten Ausführungsformen in zwei Kompartimente (21;25) unterteilt. In dem ersten Kompartiment (25) befindet sich der Wirkstoff, der vorzugsweise als Flüssigkeit vorliegt, jedoch auch als Gas-Flüssigkeitsgemisch oder gasförmig vorliegen kann. In dem zweiten Kompartiment (21), das vorzugsweise ein Verdunstungsraum (21) ist, befindet sich eine mit dem Wirkstoff gesättigte Atmosphäre A*, die nach erfolgter Perforation durch den Fluidkanal (2) abgeführt werden kann. Die Kompartimente sind durch eine Trennwand (24) voneinander separiert.

Die Behältnisse haben vorzugsweise eine Seitenlänge von maximal 20 cm. Besonders bevorzugte Ausführungsformen haben eine Grundfläche von 4cm x 4cm, alternativ 3 cm x 2 cm. Die Behältnisse fassen vorzugsweise maximal 400 mL Wirkstoff, vorzugsweise als Flüssigkeit vorliegend, im ersten Kompartiment (25). Besonders bevorzugte Ausführungsformen der Behältnisse enthalten maximal 50 mL Wirkstoff, vorzugsweise als Flüssigkeit vorliegend.

Die Verbindung zwischen erstem und zweitem Kompartiment des Behältnisses wird in bevorzugten Ausführungsformen über ein Verbindungsglied, vorzugsweise ein Verdunstungselement (20), hergestellt, das den Wirkstoff aus dem ersten Kompartiment (25) in das zweite Kompartiment des Behältnisses (21) weiterleitet.

Vorzugsweise besteht dieses Verbindungsglied (20) aus Stoff, Wolle oder einem stoffartigen bzw. wollartigen Material, das Flüssigkeit aufnehmen kann und z.B. über Adhäsionskräfte in das zweite Kompartiment (21) weiterleiten kann. In dem zweiten Kompartiment (21) verdunstet der Wirkstoff (23) aus dem Verdunstungselement (20), bis eine gesättigte Atmosphäre in dem zweiten Kompartiment (21) erreicht ist. Vorzugsweise besteht dieses Verbindungsglied (20: Verdunstungselement) aus Vlies oder Stoff.

Wenn sich die Vorrichtung zur Erzeugung eines duftstoffbeladenen Fluidstromes im Betrieb befindet, wird die gesättigte Atmosphäre A* aus dem zweiten Kompartiment (21) an die Umgebungsluft abgegeben. Der im Verbindungsglied befindliche Wirkstoff (23) verdunstet im Anschluss an die Abgabe der gesättigten Atmosphäre A* wieder in dem zweiten Kompartiment (21), so dass sich wieder eine mit Wirkstoff gesättigte Atmosphäre A* in dem zweiten Kompartiment (21) des Behältnisses (16) bildet. Das Verbindungsglied (20) ist vorzugsweise permanent in dem ersten Kompartiment (25) in Kontakt mit dem Wirkstoff (23), vorzugsweise taucht das Verbindungsglied in einen flüssigen Wirkstoff ein. Auch Restmengen des Wirkstoffs kann das Verbindungselement aus dem ersten Kompartiment in das zweite Kompartiment leiten, so dass das erste Kompartiment im Verlaufe der Nutzung des Behältnisses vollständig geleert wird.

Die Verbindung zwischen erstem (21) und zweitem (25) Kompartiment des Behältnisses kann ferner auch über andere Verbindungsarten wie gasdurchlässige Membranen hergestellt sein.

Des Weiteren weist bei besonderen Ausführungsformen das Behältnis nur ein Kompartiment auf. Die gesättigte Atmosphäre bildet sich bei diesen Ausführungsformen zum Beispiel dadurch, dass der Wirkstoff retardiert aus einer Flüssigkeit oder einem Feststoff freigesetzt wird.

Die Behältnisse werden vorzugsweise durch mechanischen Einfluss perforiert. Es sind jedoch auch andere Perforationsmethoden anwendbar.

Die Behältnisse sind hygienisch einwandfrei und lassen sich leicht aus dem Hohlraum herausnehmen und problemlos entsorgen.

Die verschliessende Folie (17) ist vorzugsweise oberseitig am Behältnis angebracht. Die Perforationsmittel (18a, 18b) durchtrennen die Folie (17) präzise an den Perforationsstellen, vorzugsweise ohne weitere Teile der Folie zu beschädigen. Vorzugsweise besteht die verschliessende Folie aus Metall oder Kunststoff.

Fluide gemäss Anspruch 1 sind als Luft-Wirkstoffgemische zu verstehen. Der Fluidkanal (2) ist dementsprechend ein Kanal, der derartige Luft-Wirkstoffgemische zur Austrittsöffnung (9) leitet. Die ersten Perforationsmittel (18a), die sich am Ende des Fluidkanals (8) befinden, bilden vorzugsweise gleichzeitig die hohlraumseitigen Enden des Fluidkanals aus. Ferner umfasst der Fluidkanal ein Mittelteil und eine Austrittsöffnung (9). Eine verlustfreie Weiterleitung der gesättigten Atmosphäre A* aus dem Behältnis (16) durch den Fluidkanal zur Austrittsöffnung (9) wird dadurch möglich. Die am Gehäuse angeordnete Austrittsöffnung des Fluidkanals (2) ist durch einen Mechanismus verschliessbar. Vorzugsweise ist der Fluidbeförderer (10) im Fluidkanal (2) angeordnet.

Der Luftkanal (3) umfasst eine Eintrittsöffnung (13), durch die die Umgebungsluft einströmt, ein Mittelteil und eine in den Hohlraum mündende Austrittsöffnung (14). An den Enden der Austrittsöffnung (15) des Luftkanals befinden sich die zweiten Perforationsmittel (18b). In besonders bevorzugten Ausführungsformen bilden die Perforationsmittel (18b) das hohlraumseitige Ende des Luftkanals (3).

Der Hohlraum (4), der Fluidkanal (2) und der Fluidbeförderer (10) bilden vorzugsweise im Verbund eine Leitung zu der Austrittsöffnung (9) des Fluidkanals (2) aus, wenn die Vorrichtung gemäss Anspruch 1 die Position F.2) gemäss Anspruch 1 einnimmt.

Bei Vorhandensein eines Luftkanals (3) können Luftkanal (3), Hohlraum (4), Fluidkanal (2) und Fluidbeförderer (10) im Verbund eine Leitung zu der am Gehäuse angeordneten Austrittsöffnung (9) des Fluidkanals (2) ausbilden wenn die Vorrichtung die Position F.2) gemäss Anspruch 1 einnimmt.

Bei Platzierung eines Behältnisses (16) im Hohlraum (4), das bei Schliessen des Gehäuses perforiert wird (Zustand F.2) gemäss Anspruch 2), wird das Behältnis Teil der Leitung zur Austrittsöffnung (9) des Fluidkanals am Gehäuse. Durch diese gemeinsam ausgebildete Leitung strömt der Luftstrom q*, angetrieben durch den Fluidbeförderer (10), zum Ausgangskanal.

Der Fluidbeförderer ist vorzugsweise ein Ventilator. Bei der Inbetriebnahme des Geräts arbeitet er vorzugsweise 3 bis 20 Sekunden. Der von der Vorrichtung abgegebene Volumenstrom q* ist minimal ein Teil des Volumens des Kompartiments (21). Maximal ist es ein Vielfaches des Volumens des zweiten Kompartiments (21). Der Fluidbeförderer arbeitet mit einer Kraft, die der Grösse des Gerätes angemessen ist. Der Ventilator weist vorzugsweise ein Laufrad mit einer Vielzahl von Elementen auf. Der Fluidkanal (2) kann je nach Einstellung des Ventilators durch ein Element des Ventilators verschlossen oder geöffnet sein. Wenn der Ventilator den Fluidkanal (2) nicht verschliesst, erfolgt durch die erfindungsgemässe Vorrichtung eine Grundbeduftung der Umgebung durch geringfügig austretenden Wirkstoff. Aber auch unabhängig von der Ventilatorstellung kann bei bevorzugten Ausführungsformen eine Grundbeduftung durch den Wirkstoff (23) erfolgen, weil Kleinstmengen des Wirkstoffs an der Perforationsstelle der zweiten Perforationsmittel (18b) permanent aus dem Behältnis entweichen. Der Volumenstrom q* der gesättigten Atmosphäre im Fluidkanal lässt sich bei speziellen Ausführungsformen durch Blenden und/oder Schieber abhängig von Parametern wie Raumgrösse oder Temperatur steuern. Auf diese Weise wird der Einsatz des Wirkstoffs (23) noch effizienter gestaltet.

Der Fluidbeförderer (10) kann auch in Form einer Membranpumpe, Piezo-Förderer etc. vorliegen. Der Fluidbeförderer (10) kann ferner einen Motor (11) aufweisen, der vorzugsweise elektrisch betrieben wird. Bei Vorliegen von mehreren Fluidbeförderern (10) können auch mehrere Motoren (11) angebracht sein.

Bei Vorliegen von zwei oder mehreren Hohlräumen (4) können auch zwei oder mehrere Fluidbeförderer (10) vorliegen, aber auch ein einzelner Fluidbeförderer kann A* aus mehreren Fluidkanälen (2), vorzugsweise aus mehreren Behältnissen, zur Austrittsöffnung (9) befördern. In besonderen Ausführungsformen gibt es zwei, drei oder mehr Austrittsöffnungen (9). Bei Vorliegen von mindestens zwei Hohlräumen (4) liegen bei weiteren Ausführungen mindestens zwei Fluidbeförderer (10) vor. Bei weiteren Ausführungsformen können Fluidbeförderer (10) sowohl im Fluidkanal (2) als auch im Luftkanal (3) angeordnet sein.

Das Gehäuse (1) weist einen beweglichen Bestandteil (19) auf, welcher mit den ersten Perforationsmitteln (18a) und/oder den zweiten Perforationsmitteln (18b) mechanisch gekoppelt ist. Dieser bewegliche Bestandteil (19) ist beispielsweise über einen Klappmechanismus oder vergleichbaren mechanischen Kopplungen mit dem übrigen Gehäuse (1) verbunden. Das Öffnen des Gehäuses erfolgt über eine Bewegung des beweglichen Teiles (19) des Gehäuses, so dass die Behältnisse im Hohlraum (4) platzierbar sind.

In bevorzugten Ausführungsformen sind die hohlraumseitigen Enden (8) des Fluidkanals (2) und die daran befindlichen Perforationsmittel (18a) mit dem beweglichen Bestandteil des Gehäuses (19) verbunden, vorzugsweise mechanisch gekoppelt.

Bei mechanischer Kopplung von beweglichen Teilen des Gehäuses und den Perforationsmitteln nimmt die Vorrichtung (12) mit dem geöffneten Gehäuse die Position F.1) gemäss Anspruch 1 und das geschlossene Gehäuse entsprechend die Position F.2) gemäss Anspruch 1.

Die zweiten Perforationsmittel (18b) sind bei besonderen Ausführungsformen ebenfalls an den beweglichen Bestandteilen des Gehäuses (19) fixiert. Durch einfaches Einführen der Behältnisse und sich anschliessendes Schliessen des Gehäuses sind die im Hohlraum (4) befindlichen Behältnisse (16) perforiert und die Vorrichtung funktionsbereit. Bei besonderen Ausführungsformen befinden sich die zweiten Perforationsmittel nicht am beweglichen Teil des Gehäuses (19).

Gemäss Anspruch 1 ist bei geschlossenem Gehäuse (1) die Vorrichtung sofort einsatzbereit. Die notwendige Sättigungskonzentration A* im Behältnis liegt bereits vor der Perforation in den geschlossenen Behältnissen (16) vor, da das Verbindungselement (20), vorzugsweise als Verdunstungselement (20) ausgestaltet, permanent in die Flüssigkeit eintaucht. Bei den nach dem Stand der Technik bekannten Vorrichtungen ist es notwendig, zu warten, bis sich eine Sättigungskonzentration nach Wechsel des Verdunstungselements und der Flüssigkeit eingestellt hat.

Bei weiteren Ausführungen weist die Vorrichtung gemäss Anspruch 1 eine Montageplatte, vorzugsweise eine Schnellmontageplatte, auf. Diese Montageplatte bildet die Grundlage für die Montage des Gehäuses (1) und für die Befestigung an der Wand. Das Gehäuse kann durch eine Verschlussvorrichtung oder durch einen Einschnappmechanismus verschliessbar sein.

In bevorzugten Ausführungsformen ist der Wirkstoff (23) vorzugsweise ein parfümierender Stoff, ein Gerüche neutralisierender Stoff, ein desodorierender Stoff oder eine Mischung der aufgeführten Stoffkategorien. Der Wirkstoff liegt vorzugsweise als Flüssigkeit im Behältnis vor und verflüchtigt sich, wenn die Atmosphäre im ersten Kompartiment (20: Verdunstungsraum) nicht gesättigt ist. Der Wirkstoff (23) ist vorzugsweise ein Duftstoff. Vorzugsweise sind leicht flüchtige ätherische Öle oder vergleichbare Duftstoffe enthalten, die leicht flüchtig sind. Der Wirkstoff kann auch eine Kombination von mehreren Duftstoffen sein.

Die Vorrichtung kann ein Steuerelement (26) umfassen, das zentral die Elemente wie Fluidbeförderer (10), Blenden, Sensoren etc. zentral steuert. Das Steuerelement (26) ist beispielsweise ein Mikrokontroller.

Das Steuerelement (26) kann die parallele Nutzung von 2 Behältnissen (16) veranlassen, um einen besonders intensiven Effekt zu erzielen. Vorzugsweise koordiniert das Steuerelement (26) bei Vorliegen von zwei oder mehreren parallel geschalteten Behältnissen die Einzelnutzung der Behältnisse (konsekutiver Verbrauch der Behältnisse), um die Serviceintervalllänge zu optimieren. Das Steuerelement (26) kann auch eine Parallelnutzung von Behältnissen einstellen. Bei Vorliegen von mindestens zwei Behältnissen können durch die Parallelnutzung verschiedene können Wirkstoffe miteinander kombiniert werden. Vorzugsweise wird die Vorrichtung gemäss Anspruch 1 für 3 bis 20 Sekunden in Betrieb genommen, da sich dieser Zeitraum für die Grösse der meisten Sanitärräumlichkeiten bewährt hat. Das Verfahren erfordert nur ein Minimum an Hilfsenergie und ermöglicht einen nahezu geräuschlosen Kurzzeit-Betrieb. Ausführungsformen der Vorrichtung gemäss Anspruch 1 umfassen Sensoren, die auf Licht, Geräusche oder Bewegungen beliebiger Art reagieren und die Signale an das Steuerelement weiterleiten und somit eine Inbetriebnahme der Vorrichtung bewirken.

Weitere Ausführungsformen umfassen optische Sensoren, die eine Füllstandsmessung der Behältnisse vornehmen und dem Nutzer auf diese Weise den nächsten Servicetermin zum Austausch der Behältnisse anzeigen.

Die Vorrichtung zur Erzeugung eines duftstoffbeladenen Fluidstromes (12) lässt sich universell einsetzen, bevorzugt findet die Vorrichtung in Toiletten und Waschräumen Anwendung. Die Inbetriebnahme der Vorrichtung (12) erfolgt bedarfsorientiert. Bedarf besteht beispielsweise bei Nutzung eines Toilettenraumes durch eine Person. Sensoren erkennen durch Geräusche oder Bewegungen oder Sensoren an Türen diesen Bedarf, der zur Inbetriebnahme der Vorrichtung gemäss Anspruch 1 führt. Der Fluidbeförderer (10) schaltet sich bedarfsorientiert ein. Er kann sich auch intermittierend und/oder periodisch einschalten.

Besonders vorteilhaft ist die bedarfsorientierte Inbetriebnahme der Vorrichtung nach Anspruch 1 durch Geräuschsignale. Hierzu ist ein Geräuschsensor an der Vorrichtung angebracht. Als Detektionsgrössen gelten bei dieser Ausführung in Toiletten- und Waschräumen insbesondere das Schliessen von Türen und/oder die Betätigung einer Wasserspülung.

Die Vorrichtung kann ferner einen Energiespeicher, vorzugsweise eine Batterie, umfassen. Eine weitere Ausführungsform umfasst ein Solarelement, das sich durch Sonnenstrahlung oder künstliches Licht auflädt und ebenfalls als Energiespeicher fungiert. Das Steuerelement kontrolliert den Energiespeicher.

In den weiteren abhängigen Ansprüchen sind vorteilhafte Weiterbildungen des Erfindungsgegenstandes beschrieben.

Die Erfindung und Weiterbildungen der Erfindung werden im Folgenden anhand der teilweise schematischen Darstellungen von Ausführungsbeispielen noch näher erläutert.
Figur 1: Vorrichtung zur Erzeugung eines duftstoffbeladenen Fluidstromes in einer Querschnittsansicht von der Vorderseite mit eingesetzten Behältnissen
Figur 2: Vorrichtung zur Erzeugung eines duftstoffbeladenen Fluidstromes in einer Querschnittsansicht von der Vorderseite ohne eingesetzte Behältnisse
Figur 3: Vorrichtung zur Erzeugung eines duftstoffbeladenen Fluidstromes in einer perspektiven Querschnittsansicht von der Vorderseite mit eingesetzten Behältnissen
Figur 4: Perspektive Schrägansicht auf die Vorrichtung zur Erzeugung eines duftstoffbeladenen Fluidstromes ohne eingesetzte Behältnisse
Figur 5: Perspektive Schrägansicht auf die Vorrichtung zur Erzeugung eines duftstoffbeladenen Fluidstromes mit eingesetzten Behältnissen
Figur 6: Querschnittsansicht von der Seite durch den Hohlraum mit perforiertem Behältnis
Figur 7: Querschnittsansicht von der Seite durch den Fluidbeförderer
Figur 8: Querschnittsansicht eines Behältnisses mit zwei Kompartimenten und einem Verdunstungselement
Figur 9: Perspektive Darstellung eines Behältnisses von der Vorderseite Detailbeschreibung der Figuren:

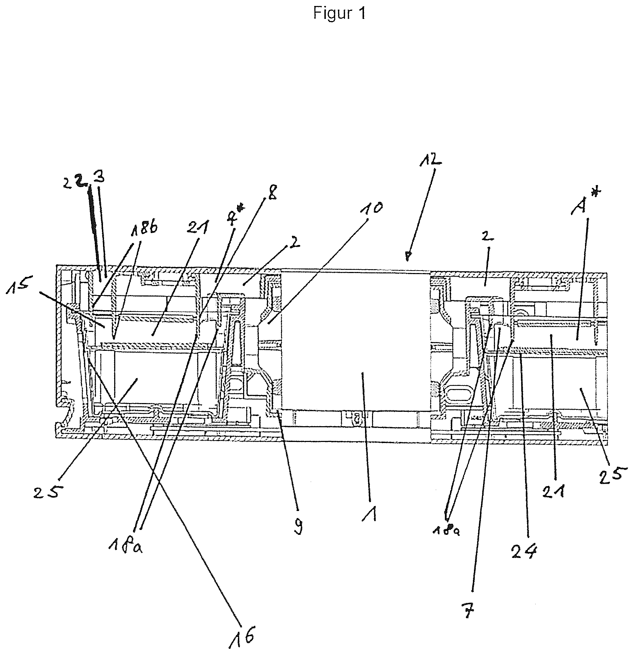

   Die Figur 1 zeigt eine Vorrichtung zur Erzeugung eines duftstoffbeladenen Fluidstromes mit zwei perforierten Behältnissen (16) in einer Querschnittsansicht. Die Position F.2) gemäss Anspruch 1 ist von dem Fluidkanal (2) und den Perforationsmitteln eingenommen. Die Vorrichtung ist im abgebildeten Zustand betriebsbereit. Die verschliessende Folie (17) des Behältnisses (16) ist durch die ersten und zweiten Perforationsmittel (18a, 18b) bündig perforiert. Die gesättigte Atmosphäre A * kann als Fluidstrom q*, angesaugt durch den im Fluidkanal (2) befindlichen Fluidbeförderer (10), aus der Vorrichtung (12) herausbefördert werden. Der Fluidbeförderer (10) ist bei besonderen Ausführungsformen zwischen 3 und 20 Sekunden in Betrieb. Zudem befindet sich an den zweiten Perforationsmitteln ein Luftkanal (3), so dass ein Druckausgleich im Behältnis während und nach der Inbetriebnahme möglich ist. Es befinden sich in der symmetrisch aufgebauten Vorrichtung jeweils zwei Fluidbeförderer (10), zwei Fluidkanäle (2), zwei Behältnisse (16), zwei Hohlräume (4) und zwei Luftkanäle (3). Der Luftkanal (3), das Behältnis (16) und der Fluidkanal (2) bilden im Verbund eine Leitung gemäss Zustand F.2) des Anspruchs 1. Deutlich sichtbar ist das Gehäuse (1), das die gesamte Vorrichtung umschliesst und einen beweglichen Bestandteil (19) aufweist. Der Vorteil dieser Anordnung liegt in der Möglichkeit, die Behältnisse parallel oder konsekutiv zu nutzen.

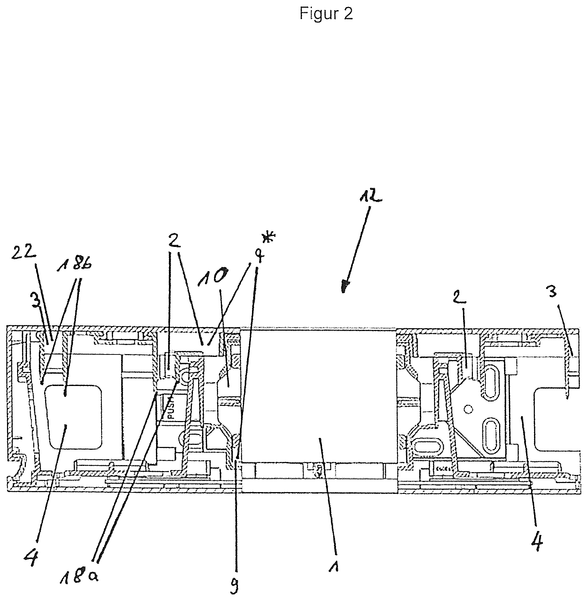

Figur 2 zeigt die in Figur 1 dargestellte Vorrichtung (12) ohne eingesetzte Behältnisse im Zustand F.2) gemäss Anspruch 1. Die Perforationsmittel (18a, 18b) ragen in den Hohlraum (4) und wären in der Lage, ein eingesetztes Behältnis zu perforieren. Luftkanal (3), Hohlraum (4), Fluidkanal (2) bilden im Verbund eine Leitung aus.

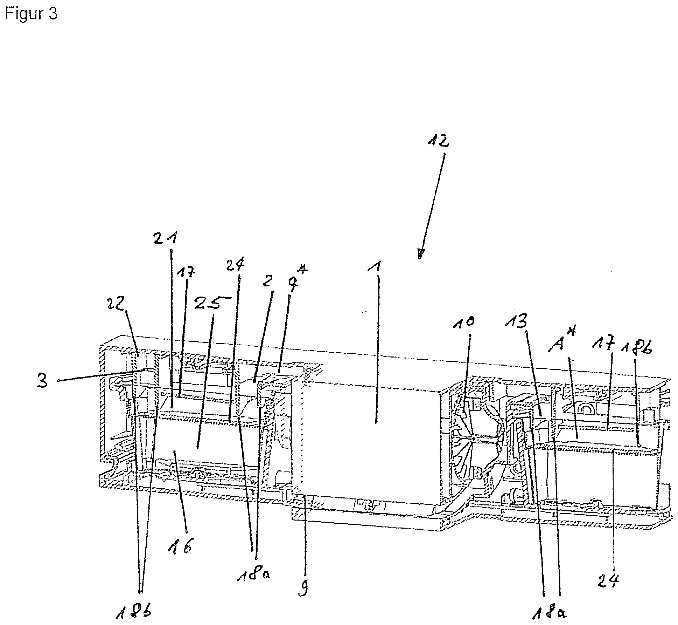

Figur 3 zeigt die Figur 1 aus einer versetzten Perspektive mit eingesetzten perforierten Behältnissen (16). Die Perforationsmittel ragen bis zur in die Nähe der Trennwand ins das zweite Kompartiment (21:Verdunstungskammer). In dieser Ansicht ist der Fluidbeförderer (10) deutlich zu erkennen, der als Ventilator ausgestaltet ist. Der Ventilator kann so eingestellt werden, dass er im Ruhezustand den Fluidkanal von der Umgebungsluft separiert.

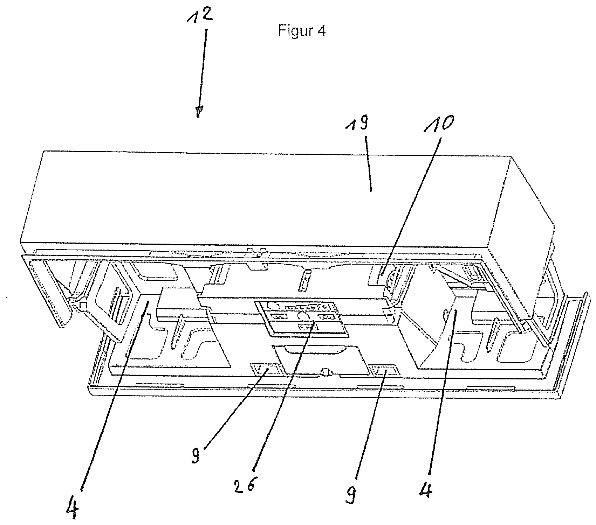

Figur 4 zeigt die Figur 1 ohne eingesetzte Behältnisse (16). Die Perforationsmittel und der Fluidkanal nehmen gemäss Anspruch 1 die Position F.1), so dass die Behältnisse in die beiden Hohlräume (4) einführbar sind. Bei dieser bevorzugten Ausführungsform sind die Perforationsmittel (18a, 18b) und der Fluidkanal (2) an das Gehäuse gekoppelt, so dass durch das Aufklappen des Gehäuses die Perforationsmittel (18a,18b) und der Fluidkanal (2) nicht mehr in den Hohlraum ragen. Der geöffnete Zustand des Gehäuses ist dargestellt, was nach Anspruch 1 dem Zustand F.1) entspricht. Ein Steuerelement (26), das sich im geschlossenen Zustand des Gehäuses innerhalb des Gehäuses befindet, ist deutlich sichtbar.

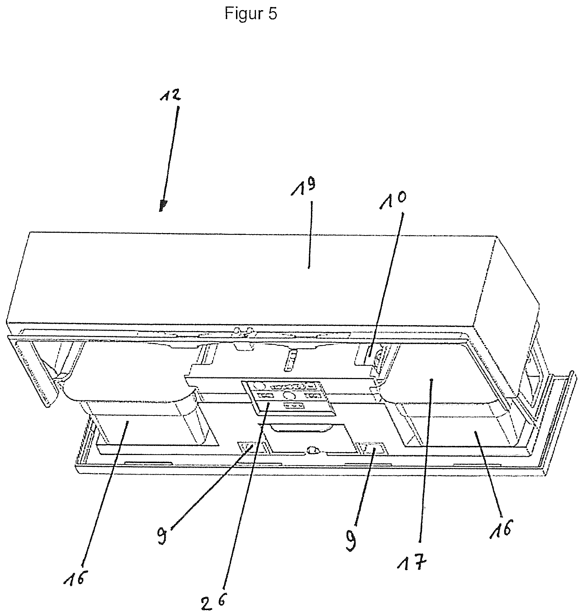

Figur 5 zeigt die Vorrichtung gemäss Figur 1 und Anspruch 1 mit geöffnetem Gehäuse (1) und eingesetzten Behältnissen (16), die verschliessende Folien aufweisen (17). Der offene Zustand des Gehäuses wird durch den beweglichen Bestandteil des Gehäuses (19) ermöglicht. Durch Herunterklappen des beweglichen Teiles (19) des Gehäuses erfolgt die Perforation der eingesetzten Behältnisse (16). An dem beweglichen Teil des Gehäuses sind Auslassungen sichtbar, damit der Fluidstrom nicht durch das Gehäuse blockiert wird. Es ist ebenfalls der Zustand F.1) nach Anspruch 1 dargestellt.

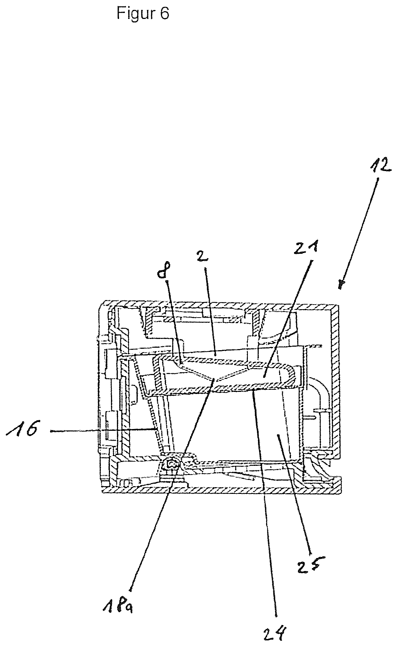

Figur 6 in einer Querschnittsansicht von der Seite die Vorrichtung mit dem perforierten Behältnis. An dem Fluidkanal (2) befinden sich die Perforationsmittel (18a). Der Fluidkanal (2) ragt in das zweite Kompartiment (21) des Behältnisses hinein und kann die gesättigte Atmosphäre A* ableiten. Sichtbar ist in dieser Ansicht die Austrittsöffnung aus dem Gehäuse (9). Das Behältnis enthält ein Verbindungselement (20:Verdunstungselement), das erstes (25) und zweites (21) Kompartiment miteinander verbindet.

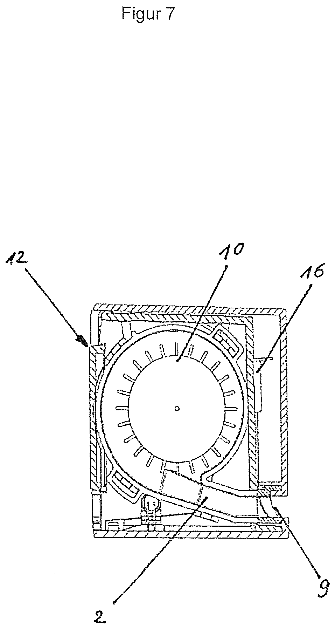

Figur 7 zeigt einen Fluidbeförderer (10) und die Austrittsöffnung (9) in einer Querschnittsansicht von der Seite. Der Fluidbeförderer (10) ist in dieser abgebildeten Ausführungsform ein Ventilator.

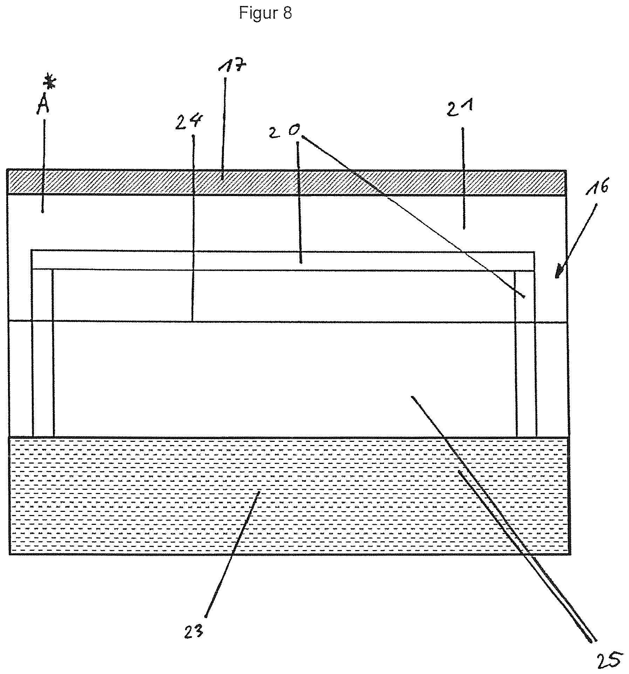

Figur 8 zeigt eine Querschnittsansicht eines Behältnisses (16) mit zwei Kompartimenten (21, 25) und einem Verdunstungselement (20). Erkennbar sind in dieser Ansicht ferner eine verschliessende Folie (17), die Trennwand (24) und der Wirkstoff als Flüssigkeit (23) im ersten Kompartiment. Das Verbindungselement (20) ist bei dieser Anordnung in der Lage, durch Adhäsionskräfte den Wirkstoff aufzunehmen und in den Verdunstungsraum (21) zu transportieren. Die Flüssigkeit kann vollständig aus dem ersten (21) in das zweite Kompartiment (25) weitergeleitet werden, weil das Verdunstungselement bis zum Boden des Behältnisses reicht.

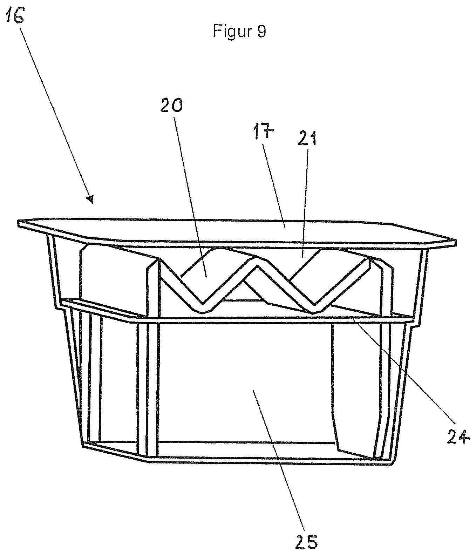

Figur 9 zeigt ein Behältnis. Das Verdunstungselement (20) ist im zweiten Kompartiment (21) gewellt, um bessere Verteilung des Wirkstoffs zu erzielen. Die Trennwand (24) wird an zwei Stellen von dem Verdunstungselement (20) unterbrochen.

## Patentansprüche

1. Vorrichtung zur Erzeugung eines duftstoffbeladenen Fluidstromes (12), umfassend:
A) einen Hohlraum (4) zur Aufnahme mindestens eines einen flüchtigen Wirkstoff (23) enthaltenden Behältnisses (16),
B) einen Fluidkanal (2), mit einer in den Hohlraum (4) mündenden Eintrittsöffnung (7), ein die Eintrittsöffnung (7) umfassendes hohlraumseitiges Ende (8) und einer Austrittsöffnung (9);
C) einen Fluidbeförderer (10), mittels welchem Fluid (q*) in Richtung der Fluidaustrittsöffnung (9) förderbar ist;
**dadurch gekennzeichnet, dass**
D) das hohlraumseitige Ende (8) des Fluidkanals (2) erste Perforationsmittel (18a) umfasst, die relativ zum Hohlraum (4) beweglich sind;
E) die Vorrichtung zweite Perforationsmittel (18b) umfasst, die relativ zum Hohlraum (4) beweglich sind;
F) wobei das hohlraumseitige Ende des Fluidkanals (8) mit den ersten Perforationsmitteln (18a) und die zweiten Perforationsmitteln (18b) zwei Positionen einnehmen können:
F.1) eine Position ausserhalb des Hohlraumes (4);
F.2) eine Position, bei der sie in den Hohlraum (4) hineinragen und ein im Hohlraum befindliches Behältnis (16) perforieren können; wobei
G) der Hohlraum (4) eine Verbindung zur Umgebungsluft (22) aufweist; und wobei
H) die Verbindung zur Umgebungsluft (22) einen Luftkanal (3) umfasst mit einer Eintrittsöffnung (13), einer in den Hohlraum (4) mündenden Austrittsöffnung (14) und einem die Austrittsöffnung (14) umfassenden hohlraumseitigen Ende (15), wobei das hohlraumseitige Ende (15) des Luftkanals (3) in den Hohlraum (4) hineinragt und die zweiten Perforationsmittel (18b) zur Perforierung eines Behältnisses (16) umfasst; wobei;
I) die Vorrichtung ein schliessbares Gehäuse umfasst; wobei
J) das Gehäuse einen beweglichen Bestandteil (19) umfasst und der bewegliche Bestandteil (19) des Gehäuses mit den ersten Perforationsmitteln (18a) und zweiten Perforationsmitteln (18b) mechanisch gekoppelt ist.

2. Vorrichtung zur Erzeugung eines duftstoffbeladenen Fluidstromes (12) nach Anspruch 1, **dadurch gekennzeichnet, dass** die Austrittsöffnung (9) des Fluidkanals (2) offen ist.

3. Vorrichtung zur Erzeugung eines duftstoffbeladenen Fluidstromes (12) nach Anspruch 1 oder 2, **dadurch gekennzeichnet, dass** im Zustand F.2) die ersten Perforationsmittel (18a) und zweiten Perforationsmittel (18b) in den Hohlraum (4) hineinragen.

4. Vorrichtung zur Erzeugung eines duftstoffbeladenen Fluidstromes (12) nach einem der Ansprüche 1 bis 3, **dadurch gekennzeichnet, dass** im Zustand F.2) der Hohlraum (4), der Fluidkanal (2) und der Fluidbeförderer (10) im Verbund eine Leitung zur Austrittsöffnung (9) des Fluidkanals (2) ausbilden.

5. Vorrichtung zur Erzeugung eines duftstoffbeladenen Fluidstromes (12) nach Anspruch 1, **dadurch gekennzeichnet, dass** im Zustand F.2) der Hohlraum (4), der Fluidkanal (2), der Luftkanal (3) und der Fluidbeförderer (10) im Verbund eine Leitung zur Austrittsöffnung (9) des Fluidkanals (2) ausbilden.

6. Vorrichtung zur Erzeugung eines duftstoffbeladenen Fluidstromes (12) nach einem der Ansprüche 1 bis 5, **dadurch gekennzeichnet, dass** im Hohlraum (4) mindestens ein perforierbares Behältnis (16) platziert ist, das einen flüchtigen Wirkstoff (23) beinhaltet und eine verschliessende, perforierbare Folie (17) umfasst.

7. Vorrichtung zur Erzeugung eines duftstoffbeladenen Fluidstromes (12) nach Anspruch 6, **dadurch gekennzeichnet, dass** durch Blenden und/oder Schieber der austretende Volumenstrom q* der gesättigten Atmosphäre mit verdunstetem Wirkstoff (23) aus dem Gehäuse (1) abhängig von Raumgrösse und Temperatur einstellbar ist.

8. Vorrichtung zur Erzeugung eines duftstoffbeladenen Fluidstromes (12) nach einem der Ansprüche 1 bis 7, **dadurch gekennzeichnet, dass** der Fluidbeförderer (10) im Fluidkanal (2) angeordnet ist.

9. Vorrichtung zur Erzeugung eines duftstoffbeladenen Fluidstromes (12) nach einem der Ansprüche 1 bis 8, **dadurch gekennzeichnet, dass** die ersten Perforationsmittel (18a) und zweiten Perforationsmittel (18b) zur Perforierung einer verschliessenden Folie (17) des Behältnisses (16) und zur Perforierung eines Behältnisses (16) geeignet sind.

10. Vorrichtung zur Erzeugung eines duftstoffbeladenen Fluidstromes (12) nach einem der Ansprüche 1 bis 9, **dadurch gekennzeichnet, dass** der Fluidbeförderer von einem Motor (11) angetrieben wird.

11. Vorrichtung zur Erzeugung eines duftstoffbeladenen Fluidstromes (12) nach einem der Ansprüche 6 bis 10, **dadurch gekennzeichnet, dass** das Behältnis (16) in zwei Kompartimente (21;25) unterteilt ist, wobei sich im ersten Kompartiment (25) der Wirkstoff befindet und das zweite Kompartiment (21) ein Verdunstungsraum ist, in welchem sich eine mit dem Wirkstoff gesättigte Atmosphäre A* befindet und der Verdunstungsraum (21) durch die Perforationsmittel (18a, 18b) perforierbar ist.

12. Vorrichtung zur Erzeugung eines duftstoffbeladenen Fluidstromes (12) nach einem der Ansprüche 6 bis 11, **dadurch gekennzeichnet, dass** in der Vorrichtung (12) zwei, drei, vier oder mehr Behältnisse zum Parallel- oder Konsekutivbetrieb platziert sind.

## Claims

1. Device for generating a fragrance-laden fluid stream (12), comprising:
A) a cavity (4) for receiving at least one container (16) containing a volatile active ingredient (23),
B) a fluid channel (2) with an inlet opening (7) opening into the cavity (4), a cavity-side end (8) surrounding the inlet opening (7) and an outlet opening (9);
C) a fluid conveyor (10) by means of which fluid (q*) can be conveyed in the direction of the fluid outlet opening (9);
**characterized in that**
D) the cavity-side end (8) of the fluid channel (2) comprises first perforation means (18a) which are movable relative to the cavity (4);
E) the device comprises second perforation means (18b) that are movable relative to the cavity (4);
F) wherein the cavity-side end of the fluid channel (8) with the first perforation means (18a) and the second perforation means (18b) can assume two positions:
F.1) a position outside the cavity (4);
F.2) a position in which they protrude into the cavity (4) and can perforate a container (16) located in the cavity; wherein
G) the cavity (4) has a connection to the ambient air (22); and wherein
H) the connection to the ambient air (22) comprises an air duct (3) with an inlet opening (13), an outlet opening (14) opening into the cavity (4), and a cavity-side end (15) comprising the outlet opening (14), wherein the cavity-side end (15) of the air duct (3) protrudes into the cavity (4) and comprises the second perforation means (18b) for perforating a container (16); wherein
I) the device comprises a closable housing; wherein
J) the housing comprises a movable component (19), and the movable component (19) of the housing is mechanically coupled to the first perforation means (18a) and second perforation means (18b).

2. Device for generating a fragrance-laden fluid stream (12) according to claim 1, **characterized in that** the outlet opening (9) of the fluid channel (2) is open.

3. Device for generating a fragrance-laden fluid stream (12) according to claim 1 or 2, **characterized in that**, in state F.2), the first perforation means (18a) and second perforation means (18b) protrude into the cavity (4).

4. Device for generating a fragrance-laden fluid stream (12) according to one of claims 1 to 3, **characterized in that**, in state F.2), the cavity (4), the fluid channel (2) and the fluid conveyor (10) together form a conduit to the outlet opening (9) of the fluid channel (2).

5. Device for generating a fragrance-laden fluid stream (12) according to claim 1, **characterized in that** in state F.2), the cavity (4), the fluid channel (2), the air channel (3), and the fluid conveyor (10) together form a conduit to the outlet opening (9) of the fluid channel (2).

6. Device for generating a fragrance-laden fluid stream (12) according to one of claims 1 to 5, **characterized in that** at least one perforatable container (16) is placed in the cavity (4), which contains a volatile active ingredient (23) and comprises a sealing, perforatable film (17).

7. Device for generating a fragrance-laden fluid stream (12) according to claim 6, **characterized in that** the escaping volume flow q* of the saturated atmosphere with evaporated active ingredient (23) from the housing (1) can be adjusted depending on room size and temperature by means of apertures and/or slides.

8. Device for generating a fragrance-laden fluid stream (12) according to one of claims 1 to 7, **characterized in that** the fluid conveyor (10) is arranged in the fluid channel (2).

9. Device for generating a fragrance-laden fluid stream (12) according to one of claims 1 to 8, **characterized in that** the first perforation means (18a) and second perforation means (18b) are suitable for perforating a sealing film (17) of the container (16) and for perforating a container (16).

10. Device for generating a fragrance-laden fluid stream (12) according to one of claims 1 to 9, **characterized in that** the fluid conveyor is driven by a motor (11).

11. Device for generating a fragrance-laden fluid stream (12) according to one of claims 6 to 10, **characterized in that** the container (16) is divided into two compartments (21; 25), the first compartment (25) containing the active substance and the second compartment (21) being an evaporation chamber in which there is an atmosphere A* saturated with the active substance, and the evaporation chamber (21) being perforable by the perforation means (18a, 18b).

12. Device for generating a fragrance-laden fluid stream (12) according to one of claims 6 to 11, **characterized in that** two, three, four, or more containers are placed in the device (12) for parallel or consecutive operation.

## Revendications

1. Dispositif pour générer un flux de fluide chargé de parfum (12), comprenant :
A) une cavité (4) destinée à recevoir au moins un récipient (16) contenant une substance active volatile (23),
B) un canal de fluide (2) avec une ouverture d'entrée (7) débouchant dans la cavité (4), une extrémité côté cavité (8) entourant l'ouverture d'entrée (7) et une ouverture de sortie (9) ;
C) un transporteur de fluide (10) au moyen duquel le fluide (q*) peut être transporté en direction de l'ouverture de sortie de fluide (9) ;
**caractérisé en ce que**
D) l'extrémité côté cavité (8) du canal de fluide (2) comprend des premiers moyens de perforation (18a) qui sont mobiles par rapport à la cavité (4) ;
E) le dispositif comprend des seconds moyens de perforation (18b) qui sont mobiles par rapport à la cavité (4) ;
F) l'extrémité côté cavité du canal de fluide (8) avec les premiers moyens de perforation (18a) et les seconds moyens de perforation (18b) pouvant prendre deux positions :
F.1) une position à l'extérieur de la cavité (4) ;
F.2) une position dans laquelle ils peuvent pénétrer dans la cavité (4) et perforer un récipient (16) situé dans la cavité ; dans lequel
G) la cavité (4) présente une connexion avec l'air ambiant (22) ; et
H) la connexion à l'air ambiant (22) comprend un canal d'air (3) avec une ouverture d'entrée (13), une ouverture de sortie (14) débouchant dans la cavité (4) et une extrémité côté cavité (15) comprenant l'ouverture de sortie (14), l'extrémité côté cavité (15) du canal d'air (3) s'étend dans la cavité (4) et comprend les seconds moyens de perforation (18b) pour perforer un récipient (16) ; dans lequel
l) le dispositif comprend un boîtier pouvant être fermé ; dans lequel
J) le boîtier comprend un composant mobile (19) et le composant mobile (19) du boîtier est couplé mécaniquement aux premiers moyens de perforation (18a) et aux seconds moyens de perforation (18b).

2. Dispositif pour générer un flux de fluide chargé de parfum (12) selon la revendication 1, **caractérisé en ce que** l'ouverture de sortie (9) du canal de fluide (2) est ouverte.

3. Dispositif pour générer un flux de fluide chargé de parfum (12) selon la revendication 1 ou 2, **caractérisé en ce que**, dans l'état F.2), les premiers moyens de perforation (18a) et les seconds moyens de perforation (18b) font saillie dans la cavité (4).

4. Dispositif pour générer un flux de fluide chargé de parfum (12) selon l'une des revendications 1 à 3, **caractérisé en ce que**, dans l'état F.2), la cavité (4), le canal de fluide (2) et le transporteur de fluide (10) forment ensemble un conduit vers l'ouverture de sortie (9) du canal de fluide (2).

5. Dispositif pour générer un flux de fluide chargé de parfum (12) selon la revendication 1, **caractérisé en ce que**, dans l'état F.2), la cavité (4), le canal de fluide (2), le canal d'air (3) et le transporteur de fluide (10) forment ensemble un conduit vers l'ouverture de sortie (9) du canal de fluide (2).

6. Dispositif pour générer un flux de fluide chargé de parfum (12) selon l'une des revendications 1 à 5, **caractérisé en ce qu'**au moins un récipient perforable (16) est placé dans la cavité (4), lequel contient une substance active volatile (23) et comprend un film perforable de fermeture (17).

7. Dispositif pour générer un flux de fluide chargé de parfum (12) selon la revendication 6, **caractérisé en ce que** le débit volumique q* sortant de l'atmosphère saturée en substance active évaporée (23) du boîtier (1) peut être réglé en fonction de la taille de la pièce et de la température à l'aide de diaphragmes et/ou de registres.

8. Dispositif pour générer un flux de fluide chargé de parfum (12) selon l'une des revendications 1 à 7, **caractérisé en ce que** le transporteur de fluide (10) est disposé dans le canal de fluide (2).

9. Dispositif pour générer un flux de fluide chargé de parfum (12) selon l'une des revendications 1 à 8, **caractérisé en ce que** les premiers moyens de perforation (18a) et les seconds moyens de perforation (18b) sont adaptés pour perforer un film de fermeture (17) du récipient (16) et pour perforer un récipient (16).

10. Dispositif pour générer un flux de fluide chargé de parfum (12) selon l'une des revendications 1 à 9, **caractérisé en ce que** le transporteur de fluide est entraîné par un moteur (11).

11. Dispositif pour générer un flux de fluide chargé en parfum (12) selon l'une des revendications 6 à 10, **caractérisé en ce que** le récipient (16) est divisé en deux compartiments (21 ; 25), le premier compartiment (25) contenant la substance active et le deuxième compartiment (21) étant un espace d'évaporation dans lequel se trouve une atmosphère A* saturée de la substance active, l'espace d'évaporation (21) pouvant être perforé par les moyens de perforation (18a, 18b).

12. Dispositif pour générer un flux de fluide chargé de parfum (12) selon l'une des revendications 6 à 11, **caractérisé en ce que** deux, trois, quatre ou plusieurs récipients sont placés dans le dispositif (12) pour un fonctionnement en parallèle ou consécutif.
